(19) Europäisches Patentamt
European Patent Office
Office européen des brevets

(11) **EP 3 781 266 B1**

(12) **FASCICULE DE BREVET EUROPEEN**

(45) Date de publication et mention de la délivrance du brevet:
**30.11.2022 Bulletin 2022/48**

(21) Numéro de dépôt: **19730395.1**

(22) Date de dépôt: **19.04.2019**

(51) Classification Internationale des Brevets (IPC):
**A61Q 19/08** (2006.01)   **A61K 8/97** (2017.01)
**A61K 8/9789** (2017.01)   **A61K 8/04** (2006.01)
**A61K 8/06** (2006.01)   **A61K 36/738** (2006.01)

(52) Classification Coopérative des Brevets (CPC):
**A61K 8/97; A61K 8/042; A61K 8/06; A61K 8/9789; A61K 36/738; A61Q 19/08**

(86) Numéro de dépôt international:
**PCT/FR2019/050941**

(87) Numéro de publication internationale:
**WO 2019/202279 (24.10.2019 Gazette 2019/43)**

(54) **COMPOSITION COSMÉTIQUE COMPRENANT UN EXTRAIT AQUEUX DE FRUITS DE ROSE**

KOSMETISCHE ZUSAMMENSETZUNG ENHALTEND WASSERLÖSLISCHEN ROSENEXTRAKTE

COSMETIC COMPOSITION COMPRISING AN AQUEOUS ROSE HIP EXTRACT

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **20.04.2018 FR 1853508**

(43) Date de publication de la demande:
**24.02.2021 Bulletin 2021/08**

(73) Titulaire: **L V M H RECHERCHE**
**45800 St. Jean de Braye (FR)**

(72) Inventeurs:
• **PECHER, Virginie**
**45380 La Chapelle Saint Mesmin (FR)**
• **FRANCHI, Jocelyne**
**45140 Saint Jean de la Ruelle (FR)**
• **POUJOL, Marion**
**45000 Orléans (FR)**

(74) Mandataire: **Regimbeau**
**20, rue de Chazelles**
**75847 Paris Cedex 17 (FR)**

(56) Documents cités:
**WO-A2-2007/112996     DE-A1- 19 651 428**
**SU-A1- 829 111     US-A1- 2010 303 872**

• **DATABASE GNPD [Online] MINTEL; 12 août 2010 (2010-08-12), anonymous: "Turkish Rose & White Tea Eye Cream", XP055531322, extrait de www.gnpd.com Database accession no. 1387244**
• **DATABASE GNPD [Online] MINTEL; 21 juillet 2017 (2017-07-21), anonymous: "Moisturizing Toning Scented Cream", XP055531325, extrait de www.gnpd.com Database accession no. 4970475**
• **DATABASE GNPD [Online] MINTEL; 26 mars 2009 (2009-03-26), anonymous: "Eye Witness Eye Repair Crème", XP055531326, extrait de www.gnpd.com Database accession no. 1078855**
• **DATABASE GNPD [Online] MINTEL; 5 mars 2018 (2018-03-05), anonymous: "Rose Hip Serum", XP055531327, extrait de www.gnpd.com Database accession no. 5497901**
• **"Rosa laevigata fruit extract INCI monograph ID: 23042" In: Nikitakis et AI: "International Cosmetic Ingredient Dictionary and Handbook", 31 décembre 2016 (2016-12-31), Personal Care Products Council, Washington DC, XP002793682, ISBN: 1-882621-55-7 vol. 3, page 3216, INCI Monograph ID 23042**

EP 3 781 266 B1

- "Rose Extract INCI monograph ID: 2723" In: Nikitakis, Joanne et Al: "International Cosmetic Ingredient Dictionary and Handbook", 31 décembre 2016 (2016-12-31), Personal Care Products Council, Washington DC, XP002793683, ISBN: 1-882621-55-7 vol. 3, pages 3219-3220, Rose Extract INCI monograph ID: 2723; page 3219 - page 3220
- "Rosa laevigata fruit extract BG80 (Mazuren)" In: Nikitakis Joanne and AI: "International Cosmetic Ingredient Dictionary and Handbook", 31 décembre 2016 (2016-12-31), Personal Care Products Council, Washington DC, XP002793684, ISBN: 1-882621-55-7 vol. 5, page 6891, page 6891
- "Rose Givree (LVMH)" In: Nikitakis Joanne et AI: "International Cosmetic Ingredient Dictionary and Handbook", 31 décembre 2016 (2016-12-31), Personal care Products council, Washington DC, XP002793685, ISBN: 1-882621-55-7 vol. 5, page 6893, page 6893
- "SFE Aqua Extract of Rose (Greentek 21)" In: Nikitakis joanne et AI: "International Cosmetic Ingredient Dictionary and Handbook", 31 décembre 2016 (2016-12-31), Personal Care Products Council, Washington DC, XP002793686, ISBN: 1-882621-55-7 vol. 5, page 6935, page 6935
- DATABASE GNPD [Online] MINTEL; 26 March 2009 (2009-03-26), anonymous: "Eye Witness Eye Repair Crème", XP055531326, Database accession no. 1078855
- DATABASE GNPD [Online] MINTEL; 5 March 2018 (2018-03-05), anonymous: "Rose Hip Serum", XP055531327, Database accession no. 5497901
- DATABASE GNPD [Online] MINTEL; 3 April 2017 (2017-04-03), anonymous: "Scented Liquid Talc for the Body", XP055531317, Database accession no. 4719499
- DATABASE GNPD [Online] MINTEL; 12 August 2010 (2010-08-12), anonymous: "Turkish Rose & White Tea Eye Cream", XP055531322, Database accession no. 1387244
- DATABASE GNPD [Online] MINTEL; 21 July 2017 (2017-07-21), anonymous: "Moisturizing Toning Scented Cream", XP055531325, Database accession no. 4970475

## Description

## DOMAINE DE L'INVENTION

La présente invention concerne une composition cosmétique comprenant au moins un extrait aqueux de fruits de rose, en particulier de rose Jardin de Granville®, et leur utilisation notamment pour favoriser et/ou améliorer l'apport en nutriments à la peau, renforcer la fonction barrière et densifier la matrice extracellulaire, en particulier pour la zone du contour des yeux.

## ETAT DE LA TECHNIQUE

Il est notoire que la qualité de la nutrition se reflète sur la santé de la peau et que certains désordres cutanés sont associés à des déficiences, en particulier en certains micronutriments (Park K. Role of micronutrients in skin health and function. Biomol.Ther. 2015 ; 23 ; 207-217). L'équilibre micro-nutritionnel de la peau peut en effet être affecté par des facteurs externes et/ou internes, tels que par exemple la fatigue, le stress, les effets oxydants, les expositions aux UV, et/ou la sénescence des cellules.
On observe notamment chez les sujets sensibles à la fatigue et au stress :

- un niveau d'hydratation moindre (cette différence s'accentue avec l'âge) ;
- un déficit en acides gras essentiels (oméga 3 et oméga 6) ;
- un déficit en acides gras à longue chaîne composant des céramides (moins bonne fonction barrière) ; et/ou
- une teneur moindre en squalène (altération du film hydrolipidique protecteur de la surface cutanée).

Le contour des yeux en particulier, qui est la zone la plus mobile du visage, réclame deux fois plus d'énergie aux cellules. Et la Demanderesse a identifié que la fatigue de cette zone active et fragile conduisait à un déséquilibre micro-nutritionnel participant à la perte d'éclat et la présence de poches et de cernes sous les yeux.
Ces observations mettent en évidence des déséquilibres, concernant en particulier des micronutriments.
Il est connu que ces nutriments, ou constituants nutritionnels, participent au niveau cutané notamment à l'établissement d'un environnement favorable à la reconstruction de la fonction barrière, à l'hydratation de la peau et donc, à terme, à une peau ressourcée et moins vulnérable au vieillissement prématuré.
Les micronutriments sont essentiels au développement et à la formation de la peau (organe en constante évolution), au renouvellement physiologique de l'épiderme et à l'adaptation de la peau à son environnement (rôle d'enveloppe de protection). Ils ont chacun un rôle particulier, des fonctions complémentaires qui couvrent les nombreuses facettes du métabolisme cutané (Park K. Role of micronutrients in skin health and function. Biomol.Ther. 2015, 23 : 207-217 ; Polefka T. Interaction of minerai salts with the skin : a literature survey. Int J. Cosmetic. Sci. 2012, 34 : 416-423 ; Boelsma E. Nutritiaonal skin care : health effects of micronutrients and fatty acids. Am. J. Clin. Nutr. 2001, 73 : 853-864 ; Winkler P. Minerais and the skin in Nutrition and the skin-Lessons for anti-aging, beauty and healthy skin. 2011, Apostolos Papas Editor; Chap 7: 91-109), et principalement :

- participent à la protection anti-oxydante (vitamines A, E, vitamine C, zinc, sélénium) ;
- sont essentiels aux métabolismes des lipides, glucides ou protéines (vitamines B3, B5, vitamine C pour la synthèse de collagène), à la production d'énergie (vitamine B2, magnésium) ;
- concourent au bon fonctionnement des enzymes, par l'apport d'ions métalliques nécessaires à leur activité (cuivre, manganèse, sélénium, zinc, fer) ;
- régulent la différenciation épidermique (calcium) ;
- sont impliqués dans la maintenance du potentiel de membrane, dans l'équilibre des fluides, et ont au niveau de la peau un rôle dans l'hydratation (potassium et sodium) ;
- participent au renforcement du film hydrolipidique qui aide la peau à conserver son élasticité et sa souplesse (Acides Gras Essentiels).

L'utilisation de vitamines (A, E, C) et/ou d'acides gras essentiels est connue dans la formulation de compositions cosmétiques destinées à la peau, notamment pour leur effet protecteur et/ou nutritionnel, mais il subsiste le besoin de nouveaux composés permettant de maintenir et/ou favoriser l'équilibre nutritionnel de la peau et par conséquent stimuler l'hydratation, la barrière cutanée, et/ou la régénération de la peau, en particulier pour la zone du contour des yeux.
Le contour des yeux présente des spécificités par rapport aux autres zones du visage :

- finesse de la peau pour faciliter la mobilité de cette zone (nombre de couches basales et épineuses inférieur par rapport à la peau du reste du visage) ;

- laxité (diminution de l'élasticité, des fibres élastiques, des collagènes) encore plus importante chez les peaux âgées, sèches ou photo-exposées ;
- cernes (coloration de la paupière inférieure) ; et
- poches (liquide dans les paupières, perte d'élasticité...) augmentées avec l'âge.

Par ailleurs, cette zone est riche en muscles, 22 au total dont 14 s'activent environ toutes les 10 secondes pour assurer une hydratation permanente à la cornée. Ces mouvements perpétuels détériorent plus rapidement les fibres qui deviennent moins fermes.

On cherche donc de nouveaux composés qui puissent apporter les éléments nutritifs assurant le maintien de l'hydratation et soient capables de stimuler le métabolisme de la peau, en particulier de la zone du contour des yeux. On connaissait de l'art antérieur l'utilisation d'extraits de fruits d'églantier (i.e *Rosa canina*) autrement nommés 'rose hip' en anglais, dans des compositions hydratantes ou anti-âge pour le visage.

Mais la Demanderesse a mis en évidence dans la présente invention l'effet *in vitro* d'un extrait aqueux (obtenu au moyen d'un solvant polaire) de fruits de rose distincts des fruits de rose de l'églantier (i.e *Rosa canina),* en particulier l'effet *in vitro* un extrait aqueux de fruits de rose Jardin de Granville®, sur la synthèse d'ATP participant à l'énergie des cellules de la peau, et sur la stimulation de l'expression de plusieurs gènes participant au renforcement de la fonction barrière et la densification de la matrice extracellulaire.

## RESUME DE L'INVENTION

Un premier objet de l'invention concerne une composition cosmétique pour application topique sur la peau comprenant, dans un milieu physiologiquement acceptable, au moins une quantité efficace d'au moins un extrait aqueux de fruits de rose, en particulier de la variété Evanrat ou rose Jardin de Granville®.

Un autre objet de l'invention concerne un procédé cosmétique destiné à favoriser et/ou améliorer l'apport en nutriments à la peau, favoriser et/ou améliorer la cohésion et/ou la différenciation épidermique, favoriser et/ou améliorer la fonction barrière, densifier la matrice extracellulaire et/ou diminuer sa dégradation, améliorer l'élasticité et/ou la fermeté de la peau en particulier du contour des yeux, prévenir et/ou diminuer les cernes et/ou les poches du contour des yeux, améliorer l'éclat et/ou l'homogénéité de la peau en particulier du contour des yeux, favoriser et/ou améliorer l'hydratation, et/ou prévenir et/ou diminuer la formation des rides et/ou ridules, comprenant l'application sur la peau, en particulier du visage et/ou du cou et de préférence du contour des yeux, d'une composition cosmétique telle que définie selon l'invention.

Selon un mode particulier et préféré, l'invention concerne un procédé cosmétique destiné prévenir et/ou diminuer les cernes et/ou les poches du contour des yeux, et/ou améliorer l'éclat et/ou l'homogénéité de la peau en particulier du contour des yeux, comprenant l'application sur le contour des yeux, d'une composition cosmétique telle que définie selon l'invention.

L'invention porte également sur l'utilisation cosmétique non thérapeutique d'au moins une quantité efficace d'au moins un extrait aqueux de fruits de rose selon l'invention, comme agent destiné à favoriser et/ou améliorer l'apport en nutriments à la peau, favoriser et/ou améliorer la cohésion et/ou la différenciation épidermique, favoriser et/ou améliorer la fonction barrière, densifier la matrice extracellulaire et/ou diminuer sa dégradation, améliorer l'élasticité et/ou la fermeté de la peau en particulier du contour des yeux, prévenir et/ou diminuer les cernes et/ou les poches du contour des yeux, améliorer l'éclat et/ou l'homogénéité de la peau en particulier du contour des yeux, favoriser et/ou améliorer l'hydratation, et/ou prévenir et/ou diminuer la formation des rides et/ou ridules.

Selon un mode particulier et préféré, l'invention porte sur l'utilisation cosmétique non thérapeutique d'au moins une quantité efficace d'au moins un extrait aqueux de fruits de rose selon l'invention, comme agent destiné à prévenir et/ou diminuer les cernes et/ou les poches du contour des yeux, et/ou améliorer l'éclat et/ou l'homogénéité de la peau en particulier du contour des yeux.

Par 'favoriser et/ou améliorer l'apport en nutriments à la peau', on entend favoriser l'apport en micronutriments présents dans l'extrait de fruits de rose de l'invention, tels que potassium et calcium (micronutriments) et phyto-sucres tels que fructose et glucose (macronutriments). Cet apport en énergie (synthèse ATP) aux cellules de la peau va permettre de favoriser le métabolisme des cellules de la peau.

Par 'densifier la matrice extracellulaire et/ou diminuer sa dégradation', on entend favoriser et/ou stimuler l'expression de protéines de la matrice extracellulaire (ex : protéines fibreuses de type collagène I et III et élastine) jouant un rôle essentiel dans l'architecture tissulaire et la régulation de la différenciation et la prolifération cellulaire.

Par 'favoriser l'éclat et/ou l'homogénéité de la peau eu particulier du contour des yeux', on entend notamment améliorer la microcirculation et/ou diminuer la perméabilité vasculaire de la peau pour une meilleure homogénéité du teint et une diminution des imperfections de couleur de peau (ex : cernes du contour des yeux).

**DESCRIPTION DETAILLEE DE L'INVENTION**

L'invention porte donc sur une composition cosmétique pour application topique sur la peau comprenant, dans un milieu physiologiquement acceptable, au moins une quantité efficace d'au moins un extrait aqueux de fruits de rose, en particulier de la variété Evanrat ou rose Jardin de Granville®.

L'extrait aqueux de fruits de rose est présent dans la composition cosmétique de l'invention dans une quantité efficace pour obtenir l'effet recherché.

En particulier, il est présent en une teneur allant de 0,1% à 95%, notamment de 0,5% à 80% en poids de matière première par rapport au poids total de la composition. Selon un premier mode de réalisation, il est présent en une teneur allant de 50% à 95%, en particulier de 60% à 90% en poids de matière première par rapport au poids total de la composition. Selon un autre mode particulier de réalisation, il est présent en une teneur allant de 0,1% à 20%, de préférence de 0,5% à 10%, et de préférence encore de 1% à 5% en poids de matière première par rapport au poids total de la composition. Des exemples illustratifs sont donnés ci-après.

### *Matériel végétal*

Les extraits de l'invention sont des extraits aqueux de fruits de rose. On parlera indifféremment selon l'invention d'un extrait de rose, d'un extrait de rosier ou d'un extrait en particulier de la variété Evanrat ou rose Jardin de Granville®.

L'homme du métier choisira de préférence des roses sélectionnées dont les propriétés sont préservées par un environnement et un mode d'agriculture biologique.

Dans le genre Rosa, on peut citer notamment *Rosa damascena, Rosa multiflora, Rosa centifolia, Rosa rugosa, Rosa chinensis, Rosa moschata, Rosa alba, Rosa alpina, Rosa cinnamonea, Rosa gallica, Rosa repens, Rosa rubrifolia, Rosa rubiginosa, Rosa sempervirens, Rosa spinosissima, Rosa stylosa, Rosa tomentosa, ou Rosa villosa*, à l'exclusion de l'espèce *Rosa canina* (églantier).

Selon un mode particulier et préféré, on utilisera un extrait aqueux de fruits de rose de la variété Evanrat ou rose 'Jardin de Granville®'.

Le rosier ou rose 'Jardin de Granville®' est une variété hybride proposée exclusivement par « Roses anciennes André Eve S.A.S » et protégée par Certificat d'Obtention Végétale sous le N°20110345 avec pour espèce la dénomination *Rosa L.* et pour la variété la dénomination EVANRAT. Ce rosier buisson appartient au groupe des hybrides modernes, qui, de mai à octobre, se couvre de roses en permanence, montrant ainsi un excellent caractère remontant.

Ainsi, selon un mode particulier et préféré de l'invention, la composition cosmétique selon l'invention comprend un extrait aqueux de fruits de rose de la variété Evanrat ou rose Jardin de Granville®.

Les fruits de rose selon l'invention sont distincts des fruits de l'églantier (i.e *Rosa canina)* appelés cynorrhodons ou 'rose hip' en anglais.

Les fruits de rose selon l'invention sont obtenus en laissant la fleur faner sur le rosier. On distingue trois types de fruits de rose selon leur maturité :

- les fruits 'verts' récoltés en octobre et issus d'une seconde floraison au mois de septembre ;
- les fruits 'intermédiaires' récoltés en octobre et issus d'une seconde floraison au mois d'août ; et
- les fruits 'mûrs' (couleur rouge-orangé) récoltés en octobre et issus d'une première floraison au mois de juin.

On utilisera ainsi selon l'invention des fruits de rose choisis respectivement parmi les fruits 'verts', les fruits 'intermédiaires, les fruits 'mûrs', et leurs mélanges.

Selon un mode particulier de l'invention, on utilisera les fruits 'verts'.

Selon un autre mode particulier de l'invention, on utilisera les fruits 'intermédiaires'.

Selon encore un autre mode particulier de l'invention, on utilisera les fruits 'mûrs'.

Dans le cadre de l'invention et selon un autre mode particulier, on utilisera les fruits 'intermédiaires' et les fruits 'mûrs'.

Les fruits 'verts, 'intermédiaires' et/ou 'mûrs' sont nommés dans le reste de la description 'fruits' de rose.

Lesdits fruits de rose sont récoltés généralement au mois d'octobre et immédiatement stockés à -20°C.

On peut utiliser les fruits entiers comprenant le chapeau, les poils et la pulpe, ou la pulpe seule.

Ces fruits de rose comprennent des sucres monosaccharides (fructose, glucose, saccharose), des acides organiques (acide citrique, acide malique), des polyphénols (catéchine), de la vitamine C, des acides aminés (majoritairement acide aspartique et acide glutamique), des minéraux (cendres, potassium, calcium), et des caroténoïdes.

En particulier, le calcium améliore la différenciation épidermique et renforce la structure de la peau, tandis que le potassium amplifie l'hydratation cutanée et booste l'assimilation de l'énergie. Les phytosucres (fructose, sucrose, saccharose) sont destinés à gorger les cellules cutanées d'énergie.

Les fruits de rose de l'invention peuvent être récoltés et directement congelés ; on parlera de fruits 'humides' ou fruits 'frais' congelés. Selon un autre mode de réalisation, les fruits de rose de l'invention peuvent être récoltés et séchés (lyophilisés) avant d'être congelés ; on parlera de fruits 'lyophilisés' congelés.

Selon un mode particulier et préféré, on utilisera des fruits 'humides' ou fruits 'frais' congelés, pour l'extraction.

L'extrait aqueux de fruits de rose selon l'invention est un extrait concentré en composés naturels polaires. L'extrait aqueux de fruits de rose selon l'invention est distinct d'une eau de rose. Il s'agit ici d'extrait de fruits de rose mettant en œuvre l'extraction de composés naturels de fruits de rose en présence de solvants aqueux (polaire) avec ou sans ajout de modificateur de pH, et une mise en œuvre, dans la formulation, sous la forme d'une solution ou concentré aqueux, le solvant du concentré pouvant être le solvant d'extraction et/ou un solvant additionnel.

Selon un mode particulier, l'extrait aqueux de fruits de rose selon l'invention comprend en outre en faible proportion un extrait de fleurs de rose représentant de 0,01 à 1%, de préférence de 0,01 à 0,1% de l'extrait aqueux total de rose selon l'invention. Dans ce mode de réalisation, le matériel végétal d'origine (10% de charge totale en matériel végétal) comprend des fruits de rose et des fleurs de rose dans une proportion 0.01% fleurs pour 9,99% de fruits à 1% fleurs pour 9% de fruits.

Les inventeurs ont en effet mis en évidence que les extraits aqueux de fruits de rose selon l'invention comprenaient des micronutriments et composés naturels d'intérêt pour la peau.

A titre de composés naturels polaires d'intérêt présents dans l'extrait aqueux de fruits de rose selon l'invention, on peut citer notamment :

- des sucres (glucose, fructose, éventuellement saccharose) et de la vitamine C, au cœur des processus énergétiques de la peau,
- des acides organiques (acide citrique, acide malique), des polyphénols (catéchine), porteurs d'activités anti-radicalaires,
- des minéraux, alliés des systèmes de protection et de réparation cutanée, et
- des acides aminés, source de vitalité et constituants des protéines pour la cellule.

L'extrait aqueux de fruits de rose selon l'invention peut être obtenu selon différents procédés connus de l'homme du métier et notamment ceux décrits ci-après.

Selon un mode particulier et préféré, on utilisera comme matériel végétal dans les procédés d'extraction adaptés à l'invention décrits ci-après, des fruits de la variété Evanrat, de préférence la rose Jardin de Granville®, de préférence des fruits frais directement congelés à -20°C.

### *Extrait aqueux de fruits de rose*

On parlera indifféremment d'extrait aqueux ou d'extrait polaire ou d'extrait hydrophile de rose dans la description. L'extrait aqueux de fruits de rose est obtenu au moyen d'un solvant polaire cosmétiquement acceptable.

Par 'extrait aqueux de rose' selon l'invention, on comprend notamment que les composés polaires (hydrophiles) des fruits de rose se sont solubilisés et/ou ont été extraits dans un solvant polaire.

Avantageusement, l'extrait de l'invention est obtenu par 'Eco extraction' basée sur la découverte et la conception de procédés d'extraction permettant de réduire la consommation énergétique, mais aussi l'utilisation de solvants alternatifs et des ressources végétales renouvelables, tout en garantissant un produit/extrait sur et de qualité. L'Eco extraction privilégie 6 grands principes (Farid Chemat, Dunod, 2010, Eco-Extraction du végétal) :

- Favoriser l'innovation par la sélection variétale et l'utilisation de ressources végétales renouvelables,

- Privilégier les solvants alternatifs dits solvants verts et principalement ceux issus des agro-ressources,

- Réduire la consommation énergétique par l'assistance des technologies innovantes et favoriser la récupération d'énergie,

- Favoriser la création de coproduits au lieu de déchets pour intégrer la voie de la bio ou agro-raffinerie,

- Réduire les opérations unitaires grâce à l'innovation technologique et favoriser les procédés surs, robustes et contrôlés, et

- Privilégier un produit non dénaturé, biodégradable, sans contaminants et surtout porteur de valeurs « eco-Extrait ».On utilisera donc avantageusement comme solvant vert polaire : l'eau.

Préalablement à l'étape d'extraction elle-même, les fruits peuvent avoir été séchés et/ou broyés. Selon un autre mode de réalisation, les fruits sont utilisés humides puis broyés.

Selon un mode particulier, les fruits sont broyés avant extraction, par exemple au moyen d'un mortier, d'un cryobroyage, d'un mixeur, d'un broyeur traditionnel ou d'un centrifugeur selon les méthodes connues de l'homme du métier.

L'extrait peut être préparé par différents procédés d'extraction connus de l'homme du métier, mettant en œuvre des étapes de broyage du matériel végétal, dispersion de la matière broyée dans un solvant polaire, séparation des phases soluble et insoluble par filtration, concentration et éventuelle remise en solution.

Selon la présente invention, l'expression « solvant polaire » signifie que le solvant a une valeur d'indice de Polarité ou paramètre de solubilité total de Hildebrand supérieur ou égal à 10. L'indice de polarité est une grandeur calculée sur la base de grandeurs thermodynamiques (de solubilité et de changement d'état) qui met en évidence le caractère plus ou moins polaire d'une molécule. A titre d'exemples, les solvants suivants ont un paramètre de solubilité total de Hildebrand de :

- 7,3 pour l'hexane (solvant apolaire),
- 11,6 pour le butylène glycol, 12,7 pour l'éthanol et 16,5 pour le glycérol (solvants polaires),
- 23,4 pour l'eau $H_2O$ (solvant polaire).

Les solvants polaires préférés sont ceux constitués d'un composé comprenant au moins une liaison covalente polaire de type O-H. A titre de solvant polaire particulièrement préféré, on choisit un solvant ou un mélange de solvants choisi(s) parmi l'eau, les alcools en $C_1$-$C_4$, tel que l'éthanol, les glycols, tels que l'éthylène glycol, le glycérol, le butylène glycol et le propylène glycol, et leurs mélanges. Avantageusement, on utilisera l'eau, l'éthanol ou leur mélange. Avantageusement, on utilisera l'eau comme solvant d'extraction (éco extraction). En particulier, on extrait le matériel végétal à l'aide d'un solvant constitué d'eau, avec ou sans modificateur de pH (ex : acide organique, acide citrique, acide chlorhydrique), sans autre solvant additionnel, dans un rapport 10g de matériel végétal pour 100g d'eau (on parlera de 10% de charge en matériel végétal). La charge en matériel végétal peut aller de 5 à 30%, de préférence 10%.

L'extraction peut être menée à chaud par reflux ou bien par macération à température ambiante.

L'extraction peut être faite à l'aide de techniques physicochimiques bien connues telles que notamment les ultrasons, micro-ondes, extrusion, champ électrique pulsé, et/ou cryo-extraction.

Dans le cas d'une extraction aux ultrasons, la durée de l'extraction pourra aller notamment de 2 à 10 minutes en particulier 5 minutes.

Dans le cas d'une extraction mettant en œuvre une autre technologie parmi celles précitées, la durée pourra aller généralement de 1h à 10h, notamment 2 à 6h, voire 4h, notamment à 60°C.

Le procédé d'extraction comprend avantageusement une étape de filtration visant à séparer la phase liquide du matériel végétal épuisé. L'homme du métier choisira des tamis de filtration adaptés, en particulier avec des diamètres de filtration de 0,1μm à 0,5μm, notamment de 0,2 à 0,3μm.

On peut reproduire le cycle d'extraction puis filtration, plusieurs fois afin d'épuiser le matériel végétal des substances ayant une affinité pour le solvant d'extraction Le procédé d'extraction peut en outre être complété par une étape d'élimination partielle ou totale des solvants d'extraction.

On peut avantageusement concentrer l'extrait en éliminant une partie du solvant ou du mélange du solvant d'extraction.

On peut ainsi obtenir un concentré aqueux dépourvu d'une quantité significative de solvants organiques ou bien encore, en éliminant tout le solvant d'extraction, obtenir un résidu sec. De façon alternative, le produit de l'étape d'extraction peut être, lyophilisé ou atomisé pour se présenter sous la forme d'une poudre.

Selon un mode particulièrement préféré, l'extrait aqueux de fruits de rose selon l'invention est obtenu selon un procédé permettant d'augmenter le rendement d'extraction et d'enrichir l'extrait en produits hydrosolubles habituellement contenus dans les sucs de plantes, notamment des sucres, des sels minéraux, des protéines, bénéfiques pour la micro-nutrition de la peau.

Ainsi selon un mode particulier et préféré de l'invention, l'extrait aqueux de fruits de rose est obtenu selon le procédé d'extraction comprenant les étapes suivantes :

   a) récolte des fruits de rose frais, éventuellement stockés à -20°C,
   b) broyage des fruits
   c) mélange de 5 à 30%, notamment 10% de matériel végétal dans de l'eau de préférence acidifiée (ex : acide citrique) à une température allant notamment de 10 à 60°C, en particulier 30°C
   d) montée progressive en température du mélange, en particulier de 30 à 100°C, notamment jusqu'à 50 à 70°C, voire jusqu'à 60°C
   e) extraction éventuellement sous agitation,
   f) séparation liquide/solide,

g) optionnellement ajout de conservateurs,

h) filtrations, en particulier jusqu'à 0,22μm,

i) optionnellement conditionnement, le cas échéant sous azote,

j) optionnellement stockage à +4°C.

Les fruits de rose sont choisis parmi des fruits 'verts', des fruits 'intermédiaires', des fruits 'mûrs' et leurs mélanges.

Selon un mode particulier, on utilise des fruits 'verts'.

Selon un autre mode on utilise des fruits 'intermédiaires' et/ou 'mûrs'.

Comme conservateurs à l'étape g), on peut citer notamment le benzoate de sodium, le sorbate de potassium, le phénoxyéthanol et leurs mélanges.

Selon un mode particulier et préféré, l'extrait aqueux de fruits de rose selon l'invention est un extrait aqueux de fruits de la variété Evanrat ou Rose Jardin de Granville® obtenu selon le procédé d'extraction décrit précédemment, autrement nommé 'Extrait aqueux de fruits de rose' et comprend de 1 à 2 % en poids de matière sèche (matière active) d'extrait de fruits de rose, 97.5% à 98.5% d'eau.

Selon un mode particulier et préféré, l'extrait aqueux de fruits de rose selon l'invention, est un extrait aqueux de fruits de la variété Evanrat ou Rose Jardin de Granville® obtenu selon le procédé d'extraction décrit précédemment, autrement nommé 'Extrait aqueux de fruits de rose' dans les exemples illustratifs décrits ci-après, et comprend de 1 à 2 % en poids de matière sèche (matière active) d'extrait de fruits de rose, 97.5% à 98.5% d'eau, 0.5% d'acide citrique, et qs de conservateurs.

Selon un mode particulier, l'extrait aqueux de fruits de rose comprend de 1 à 2 % en poids de matière sèche (matière active) d'extrait de fruits de rose et de fleurs de rose (dans un ratio massique 0,01 fleurs pour 9,99 fruits), 97.5% à 98.5% d'eau, 0.5% d'acide citrique, et qs de conservateurs.

Selon un mode particulier et préféré de l'invention, la composition cosmétique selon l'invention est caractérisée en ce que l'extrait aqueux de fruits de rose comprend un extrait de fruits de rose dans un solvant polaire, en particulier dans un ratio pondéral de 10 :90 (extrait végétal : solvant polaire) L'extrait aqueux de fruits rose selon l'invention, en particulier obtenu selon le procédé décrit précédemment, comprend avantageusement des micronutriments (calcium, potassium) et des macro-nutriments tels que les phyto sucres (glucose et fructose, et éventuellement sacharrose).

Selon un mode particulier et préféré, l'extrait aqueux de fruits rose selon l'invention en particulier de la variété Evanrat ou rose Jardin de Granville®, comprend une teneur totale en minéraux allant de 300 à 1000ppm, en particulier de 400 à 800 ppm et comprenant notamment du calcium et du potassium.

La Demanderesse a montré que la composition minérale (calcium + potassium) de l'extrait aqueux de fruits de rose selon l'invention était beaucoup plus importante que celle d'un extrait aqueux de fleurs de rose 'Cryoextrait' décrit ci-après (610ppm vs 220 ppm).

Selon un mode particulier et préféré, l'extrait aqueux de fruits rose selon l'invention, en particulier de la variété Evanrat ou rose Jardin de Granville®, comprend une teneur en sucres totaux allant de 1 à 3%, et comprenant notamment du fructose et du glucose et éventuellement du saccharose.

La Demanderesse a également montré que l'extrait aqueux de fruits de rose selon l'invention riche en phyto-sucres, appliqué à 0.3% sur des kératinocytes en culture, permettait d'obtenir une augmentation de la synthèse d'ATP de +21%.

La teneur en extrait aqueux de fruits de rose dans la composition cosmétique finale ira généralement de 0,1% à 95%, notamment de 0,5% à 80% en poids de matière première par rapport au poids total de la composition. Selon un premier mode de réalisation, il est présent en une teneur allant de 50% à 95%, en particulier de 60% à 90% en poids de matière première par rapport au poids total de la composition. Selon un autre mode particulier de réalisation, il est présent en une teneur allant de 0,1% à 20%, de préférence de 0,5% à 10%, et de préférence encore de 1% à 5% en poids de matière première par rapport au poids total de la composition..

Pour une matière première comprenant 1% en poids d'extrait sec de fruits de rose, cela équivaut à 0.001% à 0,02% en poids, notamment de 0,005% à 0,01%, et selon un mode particulier de 0,01% à 0,05% en poids de matière sèche (active) par rapport au poids total de la composition.

### *Galénique*

La composition cosmétique de l'invention peut se présenter sous toute forme galénique adaptée à une application topique sur la peau, par exemple un sérum, une émulsion huile-dans-eau, une émulsion eau-dans-huile, une émulsion multiple, ou un gel aqueux.

La composition est préférentiellement destinée à être appliquée sur le visage et se présente par exemple sous la forme d'une lotion, d'une crème de soin, d'un sérum, d'un fluide pour le visage, d'un gel de soin pour le visage et en particulier le contour des yeux. Selon un mode particulier, la composition est destinée à être appliquée sur le contour des yeux et se présente sous la forme d'un sérum, d'une crème ou d'un gel de contour des yeux, préférentiellement d'un gel aqueux de contour des yeux.

Selon un mode particulier, la composition cosmétique selon l'invention est sous la forme d'un gel aqueux pour le contour des yeux. Cette galénique sous forme de gel est particulièrement adaptée au contour des yeux, puisqu'elle confère un effet frais favorable à la décongestion des poches et des cernes, effet frais qui peut-être amplifié par le contact de l'applicateur si la composition de l'invention est appliquée sur la peau avec un applicateur tel que décrit ci-après.

La phase aqueuse représente généralement de 1 à 99% en poids, par rapport au poids total de ladite composition.

*Phase aqueuse*

La phase aqueuse de la composition selon l'invention comprend de l'eau et éventuellement un solvant hydrosoluble.

On entend par 'solvant hydrosoluble' selon l'invention, un composé liquide à température ambiante et miscible à l'eau (miscibilité dans l'eau supérieure à 50 % en poids à 25 °C et pression atmosphérique). On peut citer notamment :

- les mono-alcools inférieurs en $C_1$-$C_5$ tels que l'éthanol, l'isopropanol et leurs mélanges ;
- les glycols en $C_2$-$C_8$ tels que l'éthylène glycol, le propylène glycol, le 1,3-butylène glycol, le dipropylène glycol, et leurs mélanges;
- les polyols en $C_2$-$C_{32}$ tels que les polyglycérols, les polyéthylènes glycols, et leurs mélanges,

et leurs mélanges.

Elle peut comprendre également des gélifiants hydrophiles, des antioxydants, des conservateurs et leurs mélanges.

A titre de gélifiants hydrophiles, on peut citer notamment les polymères d'acide acrylique tels que ceux de Nom INCI Carbomer ou de référence commerciale Carbopol®, les copolymères d'acides acrylique et méthacrylique, les polymères carboxyvinyliques, les épaississants associatifs de type acrylique ou polyuréthane, les gélifiants polysaccharidiques tels que les alginates, les gommes naturelles ou modifiées telles que les gommes de xanthane, les gommes de carraghénanes, les gommes d'agar, les gommes de guar, les gommes de gellane, les chitosans , les mannanes, les pullulanes, les dérivés de la cellulose, la gélatine, les pectines, les gélifiants minéraux tels que les bentones ou les silices modifiées, et leurs mélanges.

Par gomme naturelle ou modifiée on entend un polysaccharide éventuellement modifié qui s'hydrate en milieu aqueux pour former une solution visqueuse ou une dispersion. Parmi les gommes naturelles on inclut les extraits d'algues, les exsudats de plantes et les gommes extraites de graines ou de racines végétales et celles obtenues par fermentation microbiologique. Les gommes modifiées ou semi-synthétiques comprennent les dérivés de la cellulose et de l'amidon et, de façon générale, les dérivés de toutes les gommes naturelles. Selon un mode particulier, la composition cosmétique de l'invention comprend une phase aqueuse gélifiée, notamment par la présence d'au moins un polymère acrylique, en particulier un polymère d'acide acrylique ou copolymère d'acide acrylique et méthacrylique.

Comme exemples de polymères acryliques, on peut citer notament :

- le polymère de nom INCI 'carboxyvinylpolymer' commercialisé sous la dénomination Carbopol 980 ;
- le polymère de nom INCI 'hydroxyethyl acrylate/ sodium acryloyldimethyl taurate copolymer' commercialisé sous la dénomination 'Sepinov™ EMT 10' par la société SEPPIC;
- le polymère de nom INCI 'sodium acrylate copolymer and lecithin' commercialisé sous la dénomination Lecigel™ par la société Lucas Meyer Cosmetics,

et leurs mélanges.

Selon un mode particulier et préféré, on utilisera un copolymère d'acide acrylique et méthacrylique, en particulier le polymère de nom INCI 'sodium acrylate copolymer and lecithin' commercialisé sous la dénomination Lecigel™ par la société Lucas Meyer Cosmetics,

Selon un autre mode particulier, on utilisera le polymère de nom INCI 'hydroxyethyl acrylate/ sodium acryloyldimethyl taurate copolymer' commercialisé sous la dénomination 'Sepinov™ EMT 10' par la société SEPPIC.

La teneur en gélifiant(s) hydrophile(s) dans la composition cosmétique de l'invention ira généralement de 0,5 à 5%, notamment de 0,7 à 4%, de préférence de 0,9 à 3% et de préférence encore de 1 à 2% en poids par rapport au poids total de ladite composition.

*Phase grasse ou huileuse*

La composition cosmétique de l'invention peut comprendre en outre une phase grasse (corps gras solides) ou huileuse.

On entend par « phase huileuse » une huile ou un mélange d'huiles miscibles entre elles. Par « huile », on entend, au sens de l'invention, un corps gras, non soluble dans l'eau, liquide à 25°C et 0,1 MPa, et de préférence non volatile ayant une pression de vapeur, à 25°C et 0,1 MPa, non nulle inférieure à 2,6 Pa, de préférence inférieure à 0,13 Pa.

Une phase huileuse selon l'invention peut comprendre des huiles hydrocarbonées, siliconées, fluorées ou non, et leurs mélanges.

Ces huiles peuvent être volatiles ou non volatiles, végétale, minérale ou synthétique.

Par 'huile hydrocarbonée', on entend selon l'invention une huile contenant principalement des atomes d'hydrogène et de carbone.

Par 'huile siliconée', on entend selon l'invention une huile comprenant au moins un atome de silicium, et notamment au moins un groupe Si-O.

Par 'huile fluorée', on entend selon l'invention une huile comprenant au moins un atome de fluor.

Comme huiles non volatiles hydrocarbonées, on peut citer notamment les huiles hydrocarbonées, les huiles hydrocarbonées d'origine végétale, les éthers de synthèse en $C_{10}$-$C_{40}$, les esters de synthèse en $C_{10}$-$C_{40}$, les alcools gras en $C_{12}$-$C_{26}$, les acides gras supérieurs en $C_{12}$-$C_{22}$, et leurs mélanges.

Comme huiles non volatiles siliconées, on peut citer notamment les huiles siliconées phénylées, les huiles siliconées non phénylées, et leurs mélanges.

Les huiles pourront être présentes dans la composition de l'invention en une teneur allant de 0,01 à 95 % en poids par rapport au poids total de la composition. Selon un mode particulier, les huiles seront présentes en une teneur inférieure ou égale à 10%, notamment inférieure ou égale à 5%, voire inférieure ou égale à 1% en poids par rapport au poids total de la composition. Selon un mode particulier, la composition cosmétique de l'invention ne comprend pas d'huiles, en dehors des éventuels composés lipophiles apportés par les ingrédients de la composition tels que la lécithine (phospholipide) apportée par le gélifiant Lecigel™.

La phase grasse ou huileuse peut comprendre également des gélifiants lipophiles, des polymères filmogènes, des tensioactifs, des antioxydants et leurs mélanges.

Selon un mode particulier, la composition cosmétique de l'invention est sous la forme d'un gel aqueux comprenant une phase aqueuse, au moins un gélifiant hydrophile choisi parmi les polymères d'acides acryliques, les polysaccharides et leurs mélanges.

Selon un mode particulier, la composition cosmétique est sous la forme d'un gel aqueux destiné notamment au contour des yeux comprenant au moins :

- un extrait aqueux de fruits de rose selon l'invention, en particulier un extrait aqueux de fruits de la variété Evanrat ou Rose Jardin de Granville® tel que décrit précédemment, notamment en une teneur allant de 1 à 10% en poids par rapport au poids total de la composition ;
- une phase aqueuse représentant de 80% à 99% en poids de la composition et comprenant au moins un gélifiant hydrophile, choisi notamment parmi les polymères d'acides acryliques, les polysaccharides, et leurs mélanges, et
- une phase huileuse représentant moins de 2% en poids de la composition.

Selon un mode particulier, la composition cosmétique de l'invention ne comprend pas d'autre extrait de rose que des extraits de rose de la variété Evanrat ou Rose Jardin de Granville®.

Selon un mode particulier, la composition cosmétique de l'invention comprend au moins un autre extrait de rose de la variété Evanrat ou Rose Jardin de Granville®, choisi parmi un extrait aqueux de fleurs de rose, un extrait huileux de fleurs de rose, et leur mélange, et de préférence :

- un extrait aqueux de fleurs de rose de la variété Evanrat ou Rose Jardin de Granville® obtenu selon le procédé de cryo-extraction décrit dans la demande EP0425391; en particulier un extrait comprenant 0,5% de matière sèche dans 99.5% d'un mélange d'eau/glycérol (nommé 'Cryoextrait' dans les exemples illustratifs) et
- un extrait huileux de fleurs de rose de la variété Evanrat ou Rose Jardin de Granville® obtenu selon le procédé d'enfleurage dynamique tel que décrit dans la demande décrit dans la demande WO2010/112760; en particulier un extrait comprenant de 0,5% à 1,5% de matière sèche dans 98.5-99.5% d'huile de tournesol oléique désodorisée bio (nommé 'Satin oil' dans les exemples illustratifs suivants).

L'ajout de ces extraits aqueux et huileux de fleurs de rose en particulier de la variété Evanrat ou Rose Jardin de Granville® permet un apport complémentaire en micronutriments, notamment en acides gras essentiels (omégas 3, 6 et 9) apportés par l'extrait huileux de fleurs de rose et un apport en minéraux (manganèse, magnésium, cuivre, zinc...)

apportés par l'extrait aqueux de fleurs de rose.

La Demanderesse a montré en outre dans une précédente étude que lesdits extraits aqueux de fleurs de rose et extrait huileux de fleurs de rose stimulent l'expression de gènes de l'horloge épidermique, et l'expression de gènes cibles de l'horloge épidermique, impliqués notamment dans le métabolisme lipidique, la barrière cutanée, la différenciation cellulaire, la communication cellulaire et/ou la cohésion cellulaire.

En particulier, l'extrait aqueux de fleurs de rose stimule l'expression des gènes horloges Cryptochrome Circadian Clock 2 CRY2, Period 1 PER1 et Period 3 PER3 et l'extrait huileux de fleurs de rose stimule l'expression du gène horloge Period 2 PER2.

L'extrait aqueux de fleurs de rose stimule l'expression des gènes Kératin 10 KRT10 et Desmoglein 1 DSG1 et l'extrait huileux de fleurs de rose stimule l'expression des gènes Ceramide-synthase 3 CERS3, Calmoduline 3 CALM3, Kératine 1 KRT1, Gap-junction alpha-1 protein GJA1/Connexin 43 Cx43 et Desmocollin 3 DSC3.

L'utilisation de ces extraits de fleurs de rose permet donc d'obtenir des effets complémentaires à ceux liés à l'extrait aqueux de fruits de rose selon l'invention.

La composition pourra en outre comprendre un parfum de rose.

**Ingrédients additionnels**

La composition de l'invention peut également comprendre tout additif usuellement utilisé en cosmétique tels que des filtres UV, des antioxydants, des parfums, des agents actifs cosmétiques, comme par exemple des émollients, des hydratants, des vitamines, des agents anti-âge, des agents éclaircissants, des charges, des nacres et leurs mélanges.

Comme agents actifs cosmétiques, elle peut comprendre des agents favorisant la microcirculation destinées à diminuer les poches sous les yeux, tels que des extraits de *Centella asiatica,* extrait de marron d'inde (*Aesculus hippocastanum*), *etc*.

Elle peut également comprendre des charges et/ou des nacres, en particulier des nacres blanches, destinées à masquer les imperfections de couleur (cernes) et illuminer le regard.

**Applicateur**

La composition cosmétique selon l'invention est appliquée avantageusement sur le contour des yeux. L'application peut être faite au doigt ou avantageusement à l'aide d'un applicateur, qui va permettre une application plus précise sur le contour de l'œil et/ou une efficacité améliorée en lien avec une meilleure pénétration des actifs cosmétiques et/ou une action mécanique complémentaire liée à la gestuelle de l'applicateur.

L'applicateur pourra comprendre une tête massante constituée d'une ou plusieurs billes identiques, dotées d'une rotation de 180 à 360°, de préférence 360°. La surface de massage est augmentée en comparaison d'une application au doigt, le drainage des poches est plus performant qu'avec un roll-on mono-bille. Associé à une technique de massage dans un mouvement de va et vient allant de la tempe à l'angle de l'œil, cet applicateur réactive la microcirculation et l'oxygénation cellulaire et amplifie l'assimilation des micronutriments de la composition cosmétique de l'invention.

**Procédé cosmétique**

L'invention concerne également un procédé cosmétique destiné à favoriser et/ou améliorer l'apport en nutriments à la peau, favoriser et/ou améliorer la cohésion et/ou la différenciation épidermique, favoriser et/ou améliorer la fonction barrière, densifier la matrice extracellulaire et/ou diminuer sa dégradation, améliorer l'élasticité et/ou la fermeté de la peau en particulier du contour des yeux, prévenir et/ou diminuer les cernes et/ou les poches du contour des yeux, améliorer l'éclat et/ou l'homogénéité de la peau en particulier du contour des yeux, favoriser et/ou améliorer l'hydratation, et/ou prévenir et/ou diminuer la formation des rides et/ou ridules, comprenant l'application sur la peau, en particulier du visage et/ou du cou et de préférence du contour des yeux, d'une composition cosmétique telle que définie selon l'invention comprenant au moins un extrait aqueux de fruits de rose selon l'invention, en particulier de la variété Evanrat ou rose Jardin de Granville®.

La composition peut être appliquée sur le corps, le visage et/ou le cou. Selon un mode particulier, la composition est appliquée sur le visage et/ou le cou. Selon un mode particulier et préféré, la composition est appliquée sur le contour des yeux.

Selon un mode particulier, l'invention concerne un procédé cosmétique destiné à densifier la matrice extracellulaire et/ou diminuer sa dégradation comprenant l'application sur le contour des yeux, d'une composition cosmétique telle que définie précédemment selon l'invention. Selon un mode particulier, l'invention concerne un procédé cosmétique destiné à améliorer l'élasticité et/ou la fermeté de la peau comprenant l'application sur le contour des yeux, d'une composition cosmétique telle que définie précédemment selon l'invention.

Selon un mode particulier et préféré, l'invention concerne un procédé cosmétique destiné à améliorer l'éclat et/ou l'homogénéité de couleur du contour des yeux comprenant l'application sur le contour des yeux, d'une composition cosmétique telle que définie précédemment selon l'invention.

Selon un mode particulier et préféré, l'invention concerne un procédé cosmétique destiné à prévenir et/ou diminuer les cernes et/ou les poches du contour des yeux comprenant l'application sur le contour des yeux, d'une composition cosmétique telle que définie précédemment selon l'invention.

De préférence, on utilisera dans le cadre de ces procédés cosmétique une composition cosmétique comprenant un extrait aqueux de fruits de rose selon l'invention, seul ou en combinaison avec d'autres extraits de fleurs de rose de la variété Evanrat ou rose Jardin de Granville® tels que décrits précédemment.

### *Utilisations cosmétiques*

L'invention porte encore sur l'utilisation cosmétique non thérapeutique d'au moins une quantité efficace d'au moins un extrait aqueux de fruits de rose selon l'invention, en particulier de la variété Evanrat ou rose Jardin de Granville®, comme agent destiné à favoriser et/ou améliorer la cohésion et/ou la différenciation épidermique, favoriser et/ou améliorer la fonction barrière, densifier la matrice extracellulaire et/ou diminuer sa dégradation, améliorer l'élasticité et/ou la fermeté de la peau en particulier du contour des yeux, prévenir et/ou diminuer les cernes et/ou les poches du contour des yeux, améliorer l'éclat et/ou l'homogénéité de la peau en particulier du contour des yeux, favoriser et/ou améliorer l'hydratation, et/ou prévenir et/ou diminuer la formation des rides et/ou ridules.

L'extrait aqueux de fruits de rose utilisé selon l'invention est tel que décrit ci-dessus.

De préférence, on utilisera un extrait aqueux de fruits de rose de la variété Evanrat ou Rose Jardin de Granville® obtenu selon le procédé de cryobroyage et extraction à chaud des fruits frais de rose Jardin de Granville®; en particulier un extrait comprenant 1% de matière sèche dans 97.5% d'eau, 0.5% d'acide citrique et qs conservateurs (nommé 'Extrait aqueux de fruits de rose' dans les exemples illustratifs).

L'invention va désormais être illustrée dans les exemples non limitatifs suivants.

### EXEMPLES

### Matériel et Méthodes

On utilise comme matériel végétal pour obtenir les extraits aqueux de rose illustrés dans ces exemples, des fruits de rose de la variété Evanrat, en particulier des fruits de rosier Jardin de Granville®, disponible chez les pépiniéristes.

### Extrait aqueux de fruits de rose selon l'invention

On utilise comme matériel végétal des fruits de rose de la variété Evanrat, en particulier des fruits de rosier Jardin de Granville® disponible chez les pépiniéristes.

L'extrait aqueux de fruits de rose est obtenu selon le procédé de cryobroyage et extraction à chaud (60°C) dans l'eau décrit ci-dessus. On obtient l'extrait aqueux de fruits de rose comprenant 2 % en poids de matière sèche (matière active), 97.5% en poids d'eau, 0.5% d'acide citrique et des conservateurs qsp100%.

D'autres extraits de rose, en particulier de la variété Evanrat, de préférence la rose Jardin de Granville® peuvent être utilisés en association avec l'extrait aqueux de fruits de rose selon l'invention pour apporter des effets complémentaires :

### • Extrait aqueux de fleurs de rose ('Cryoextrait')

On utilise comme matériel végétal des fleurs (pétales) de rose de la variété Evanrat, en particulier des fleurs de rosier Jardin de Granville® disponible chez les pépiniéristes.

L'extrait aqueux de fleur de rose est obtenu selon le procédé de cryo-extraction décrit ci-dessus, en particulier selon le procédé décrit dans la demande de brevet EP0425391. On obtient un Cryoextrait comprenant 0.5 % en poids de matière sèche (matière active), 49-50% en poids d'eau, 49% en poids de glycérol et des conservateurs qsp100%. Le nom INCI de cet extrait aqueux de rose est Water, Glycerin, Rose Extract ou Rosa Hybrid Flower Extract, Water, Glycerin.

### • Extrait huileux de fleurs de rose ('Satin Oil')

On utilise comme matériel végétal des fleurs (pétales) de rose de la variété Evanrat, en particulier des fleurs de rosier Jardin de Granville® disponible chez les pépiniéristes.

L'extrait huileux de fleur de rose est obtenu selon le procédé d'enfleurage dynamique décrit ci-dessus, en particulier selon le procédé décrit dans la demande de brevet WO2010/112760. On obtient un extrait huileux Satin Oil comprenant 0.5-1.5% en poids de matière sèche de rose (matière active), et 98.5-99.5% en poids d'huile désodorisée de tournesol oléique bio. Le nom INCI de cet extrait huileux de rose est Rose extract and Helianthus annuus (sunflower) seed oil ou Rosa Hybrid Flower Extract, Helianthus annuus (sunflower) seed oil.

On a étudié l'impact de l'extrait aqueux de fruits de rose sur l'énergie cellulaire (synthèse d'ATP), l'expression de gènes impliqués dans la cohésion et différenciation épidermique (fonction barrière), la densification de la matrice extracellulaire, et la perméabilité cellulaire.

**Exemple 1 : Effet de l'extrait aqueux de fruits de rose sur la synthèse d'ATP**

L'effet de l'extrait aqueux de fruits de rose sur la synthèse d'ATP de kératinocytes humains normaux (KHN) en culture a été testé.

Les KHN ont été traités pendant 24 heures avec :

- Extrait aqueux de fruits de rose : 0.3%
- Contrôle positif : Early Boost© de CODIF comme énergisant (extrait de *Jania rubens* riche en taurine associé à un oligofurcellaran) : 0.15%

Après 2 heures de traitement, on détecte et on mesure le niveau de production d'ATP par bioluminescence à l'aide du kit de la société Biovision commercialisé sous la dénomination ApoSENSOR™ ATP Cell Viability Bioluminescence Assay Kit, selon le protocole et les recommandations du fournisseur.

Cet essai utilise la luciférase pour catalyser la formation de lumière à partir d'ATP et de luciférine selon la réaction suivante :

$$ATP + luciférine + O_2 \rightarrow Oxyluciférine + AMP + PPi + Co_2 + lumière.$$

La lumière produite est mesurée à l'aide d'un luminomètre (Oméga, BMG) et est proportionnelle à la quantité d'ATP synthétisée.

Une courbe standard de $0.001\mu g/mL$ à $1000\mu g/mL$ est définie pour la concentration en ATP intracellulaire.

Après 2 heures de traitement, on observe que l'extrait aqueux de fruits de rose à 0.3% augmente significativement la synthèse d'ATP (+21%). Le contrôle positif donne le résultat attendu (+32%) et valide l'expérience.

**Exemple 2: Effet de l'extrait aqueux de fruits de rose sur le renforcement de la fonction barrière (différenciation et cohésion épidermique)**

Des kératinocytes humains normaux (KHN) sont mis en culture puis traités avec l'extrait aqueux de fruits de Rose décrit dans le paragraphe Matériels et Méthodes.

Après traitement des KHN, une étude par Taqman Low Density Array (TLDA) sur les gènes étudiés est effectuée à partir des ADNc obtenus après transcription inverse des ARN totaux extraits.

L'extrait aqueux de rose est testé à 0.15% et 0.31% en poids de matière première (excipient eau).

*Culture des KHN*

Les kératinocytes humains normaux sont issus d'un prélèvement cutané issu de chirurgie plastique. Les cellules sont cultivées le milieu Epilife complet à P5 avec une densité d'ensemencement de 50.000 cellules par puits, dans des plaques 12 puits. A sub-confluence, les cellules sont traitées 24 heures avec les doses d'extraits de rose décrites ci-dessus.

*Technologie TLDA (TaqMan Low Density Array)*

• Obtention des ARN totaux

Le milieu de culture des cellules est éliminé, et 250 $\mu$L de tampon de lyse RLT (fourni dans le kit Nucleospin RNA trace, Macherey-Nagel) sont ajoutés. Les cellules sont raclées à l'aide d'un *Cell Scraper* puis, le lysat cellulaire est récupéré dans une deepwell 1,2 mL (fourni_dans le kit Nucleospin RNA). Les ARN totaux sont extraits selon les protocoles définis.

Les solutions d'ARN totaux obtenues sont dosées, et leur qualité vérifiée, à l'aide d'un lecteur de microplaques, le spectrostarNANO (BMG Labtech) couplé au MicrolabSTAR. Cet appareil est relié à l'ordinateur pilotant la plateforme Robotique et possède le logiciel spécifique d'analyse des résultats (logiciel MARS). La technique nécessite une microplaque de 384 puits (LoBase), un contrôle positif (RNA 250, AM7155, Thermofisher) permettant de valider les pipetages réalisés par le robot ainsi que les valeurs générées par le lecteur spectrostarNANO.

• Synthèse des ADN complémentaires

Le kit de reverse transcription (RT) utilisé est le High Capacity Reverse Transcription Kit (Thermo Fisher). Il a été utilisé selon le protocole fourni. 500 ng d'ARN totaux sont dilués dans de l'eau pour un volume final de 25 µL. Ils sont ensuite incubés pendant 10 minutes à 25°C puis 2 heures à 37°C en présence de 25 µL de mélange réactionnel de High Capacity Reverse Transcription Kit 2X préalablement préparé comme indiqué ci-dessous. Les différentes incubations sont faites au sein du TRobot (Biométra).

Tableau 1 : Mélange réactionnel High Capacity Reverse Transcription Kit 2X pour 1 réaction

| Réactifs | RT tampon | dNTP | Primer | RNase OUT | RT | H2O |
|---|---|---|---|---|---|---|
| Volume | 5 µL | 2 µL | 5 µL | 0,5 µL | 2,5 µL | 10 µL |

• PCR-TaqMan Low Density Array

15 µL de chaque RT sont mélangés à 60 µl d'eau puis 75 µL de TaqMan Gene Expression master mix (ThermoFisher référence 4369510), contenant l'ADN polymerase, sont ajoutés. Après homogénéisation, 100 µL sont déposés sur les cartes microfluidiques contenant les sondes correspondant aux gènes testés (tableau 2 ci-dessous), ces dernières sont centrifugées puis scellées. Le CD Rom correspondant au profil des gènes déposés sur les plaques est chargé dans le logiciel SDS 2.3, précisant l'emplacement de chaque gène sur la carte. Le gène contrôle (ou « endogeneous » gene) à utiliser pour la normalisation des résultats est à indiquer avant le lancement de la PCR. Cette dernière est réalisée selon le protocole fourni par Applied Biosystems dans l'appareil ABI Prism 7900HT Sequence détection system. Les étapes de la qPCR sont 2 min à 50°C, 10 min à 94,5°C puis 30s à 97°C et 1 min à 59,7°C pour 40 cycles.

Tableau 2 : liste des gènes de la carte microfluidique.

| Gene | Symbole | Reference TaqMan | RefSeq N° d'accession GeneBank |
|---|---|---|---|
| Transglutaminase 1 | TGM1 | Hs00165929_m1 | NM_000359 |
| Small Prolin-Rich Protein 1B (Cornifine) | SPR1B | Hs00824893_m1 | NM_003125 |
| Cytokératine 1 | KRT1 | Hs00196158_m1 | NM_006121.3 |
| E-Cadhérine | CDH1 | Hs00170423_m1 | NM_001317184.1 |
| Connexine 43 | GJA1 | Hs00748445_s1 | NM_000165.4 |
| Desmogleine | DSG1 | Hs00170047_m1 | NM_ 001942.3 |
| Occludine | OCLN | Hs00170162_m1 | NM_001205254.2 |
| Claudine-1 | CLDN1 | Hs00221623_m1 | NM_021101.4 |

*Analyses statistiques*

La PCR quantitative en temps réel peut être exploitée si son efficacité est comprise entre 90% et 110%. Pour chaque échantillon, le nombre de cycles auquel apparaît le signal est déterminé par le logiciel SDS 2.3. Pour un même essai, les niveaux d'expression des transcrits d'intérêt obtenus sont normalisés par rapport à la valeur obtenue pour le gène de ménage Beta-2-microglobuline. Ce gène dont l'expression est constitutive et invariante permet de s'affranchir toutes variations induites au cours de l'expérience (dosage des ARNs totaux, pipetages, étape de réverse transcription, PCR dans l'appareillage).

Dans la méthode de RT-PCR TLDA, la quantification est effectuée en utilisant la méthode comparative de $\Delta\Delta Ct$. Les valeurs de quantification relative (RQ) obtenues correspondent au niveau d'amplitude (x fois plus ou moins que le contrôle) de l'expression par rapport à notre contrôle ici le non irradié. Le RQ est obtenu par le calcul suivant où le contrôle est égal à 1 :

$$RQ = 2^{-\Delta\Delta Ct} = 2^{-(\Delta Ct\ traité\ -\ \Delta Ct\ non\ traité)}$$

$$\Delta Ct\ traité = Ct\ gène\ cible\ traité - Ct\ gène\ de\ ménage\ traité$$

$\Delta Ct$ non traité Ct gène cible non traité - Ct gène de ménage non traité

Afin d'évaluer des variations d'activité transcriptionnelle statistiquement significative, nous utiliserons le test t de Student. Chaque condition est réalisée en triplicate (3 non traités et 3 traités dans les mêmes conditions). Le test-F de Fischer est tout d'abord appliqué en comparant les deux matrices de données. Lorsque la valeur est supérieur à $\alpha$ = 0,05 alors la variance pour le test t de Student est de 2, lorsque le test-F de Fischer est inférieur à $\alpha$ = 0,05 alors la variance sera égale à 3. Les variations transcriptionnelles retenues seront celles qui ont un test t de Student inférieur à $\alpha$ = 0,05.

Les résultats sont présentés en moyenne sur n=3. Le test t de Student a été utilisé pour comparer l'effet entre les cellules traitées et non traitées.

Les résultats sont considérés comme significatifs pour p<0,05(*) ou p<0,01 (**).

Les résultats sont présentés dans le tableau 3 suivant :

Tableau 3

| Type Gènes | Gène | Symbole | Extrait* 0.15% | Extrait* 0.31% |
|---|---|---|---|---|
| Différenciation/Barri ère cutanée/ Lipides/Des quamation | | | | |
| | Transglutaminase 1 | TGM1 | 2,148 | 2,716 |
| | Small Prolin-Rich Protein 1B (Cornifin) | SPRR1B | 1,782 | 2,272 |
| | Cytokératine 1 | KRT1 | 2,207 | 2,641 |
| Adhésion/Cohésion/ Communication | E-Cadhérine | CDH1 | - | 1,516 |
| | Connexin 43 | GJA1 | 1,489 | 2,199 |
| | Desmoglein | DSG1 | 1,832 | 3,139 |
| | Occludin | OCLN | 1,430 | 2,319 |
| | Claudin-1 | CLDN1 | - | 2,072 |
| *extrait aqueux de fruits de rose décrit dans le paragraphe matériels et méthodes | | | | |

A la dose de 0.31%, pour chaque marqueur, cela correspond à une augmentation respectivement de :

Tableau 4

| | |
|---|---|
| Transglutaminase | +172% |
| SPRR 1B * Small Small Proline-Rich Protein 1B | +127% |
| Kératine 1 | +164% |
| Cadherine 1 | +52% |
| Connexine 43 | +120% |
| Desmogléine 1 | +214% |
| Occludine | +132% |
| Claudine 1 | +107% |

Ces résultats montrent que l'extrait aqueux de fruits de rose selon l'invention stimule l'expression de gènes impliqués dans la cohésion et la différenciation épidermique.

Et l'effet de l'extrait aqueux de fruit de rose (0.31% en poids de matière première équivalent à 52.7μg/mL(/MS) stimule l'expression du gène Desmoglein 1 (DSG1) avec une augmentation +214% bien supérieure à celle obtenue dans une autre expérience pour un cryoextrait de fleurs à une dose légèrement supérieure (1% en poids de matière première équivalent à 65μg/mL(/MS)): +58%; sachant par ailleurs que ce cryoextrait n'a pas stimulé les autres gènes.

### Exemple 3 : Effet de l'extrait aqueux de fruits de rose sur la densification de la matrice extracellulaire

Des Fibroblastes humains normaux (FHN) ont été traités ou non avec l'extrait aqueux de fruits de rose selon l'invention pendant 24 heures, à la dose de 0.15% (équivalent à 25μg/ML de matière sèche). Après traitement des FHN, une étude par Taqman Low Density Array (TLDA) sur différents gènes est effectuée à partir des ADNc obtenus après transcription inverse des ARN totaux extraits des FHN.

*Culture des FHN*

Les fibroblastes sont issus d'une plastie abdominale d'un donneur féminin de 37 ans. Les cellules sont ensemencées dans du milieu DMEM 1 g/L glucose complémenté de 10 % de sérum de veau fœtal à P5 avec une densité d'ensemencement de 250.000 cellules par puits, dans des plaques 6 puits. La veille du traitement, les cellules sont cultivées dans du milieu DMEM 1 g/L glucose sans sérum de veau fœtal.

*Traitement des FHN*

A confluence, les cellules sont traitées avec l'extrait aqueux de fruits de rose, préparé extemporanément à la concentration finale d'utilisation dans du milieu DMEM 1 g/L glucose sans sérum de veau fœtal. Après 24 heures de traitement, les cellules sont récupérées afin d'en extraire les ARN totaux.

*TLDA (TaqMan Low Density Array)*

L'étude par la technologie de TLDA sur une liste de gènes est effectuée à partirdes ADNc obtenus après transcription inverse des ARN totaux extraits des FHN selon le protocole décrit à l'exemple 1.

Tableau 5 : liste des gènes de la carte microfluidique

| Gene | Symbole | Reference TaqMan | RefSeq N° d'accession GeneBank |
|---|---|---|---|
| Collagène 1 | COL1A1 | Hs00164004_m1 | NM_000088.3 |
| Collagène 3 | COL3A1 | Hs00164103_m1 | NM_000090.3 |
| Collagène 5 | COL5A1 | Hs00609088_m1 | NM_000093.4 |
| Elastine | ELN | Hs00355783_m1 | NM_000201.3 |
| Emiline 1 | Emilin1 | Hs00170878_m1 | NM_007046.3 |
| Fibrilline 1 | FBN1 | Hs00171191_m1 | NM_000138.4 |
| Lysyl oxidase like 1 | LOXL1 | Hs00173746_m1 | NM_005576.3 |
| Procollagen C endopeptidase enhancer | PCOLCE | Hs00170179_m1 | NM_002893.3 |
| Matrix Metalloprotease 3 | MMP3 | Hs00233962_m1 | NM_002422.4 |

Les résultats sont présentés dans le tableau 6 suivant :

Tableau 6

| Type Gènes | Gène | Symbole | Extrait* 0.15% | Vit C 50μg/mL (contrôle positif) |
|---|---|---|---|---|
| Synthèse-induction | Collagène 1 | COL1A1 | 16,015 | 3,471 |
| | Collagène 3 | COL3A1 | 2,818 | 2,599 |
| | Collagène 5 | COL5A1 | 12,987 | 1,911 |
| | Elastine | ELN | 15,762 | 1,016 |
| Assemblage | Emilin 1 | Emilin1 | 5,330 | 2,509 |
| | Fibrillin 1 | FBN1 | 1,947 | 0,989 |
| | Lysyl oxidase like 1 LOWL1 | LOXL1 | 2,995 | 1,202 |
| | Procollagen C endopeptidase enhancer | PCOLCE | 1,450 | 1,156 |
| Dégradation de la matrice | Matrix Metalloproeinase 3 | MMP3 | 0,679 | 1,205 |
| *extrait aqueux de fruits de rose décrit dans le paragraphe matériels et méthodes | | | | |

A la dose de 0.15%, pour chaque marqueur, cela correspond à une augmentation respectivement de :

Tableau 7

| | Extrait* 0.15% (25μg/mL) | Vit C (50μg/mL) |
|---|---|---|
| Collagène 1 | +1500% | +247% |
| Collagène 3 | +182% | +160% |
| Collagène 5 | +1200% | +91% |
| Elastine | +1476% | NS |
| Emilin 1 | +433% | +151% |
| Fibrillin 1 | +95% | NS |
| Lysyl oxidase like 1 LOWL1 | +200% | NS |
| Procollagen C endopeptidase enhancer | +45% | NS |
| Matrix Metalloproeinase 3 | -32% | NS |
| *extrait aqueux de fruits de rose décrit dans le paragraphe matériels et méthodes | | |

Ces résultats montrent que l'extrait aqueux de fruits de rose selon l'invention stimule l'expression génique de protéines de la matrice extracellulaire : Collagène I, III, V, élastine ainsi que des enzymes impliquées dans la formation des fibres : LOX (Lysyl oxidase), PECOL (Procollagen C-endopeptidase enhancer), et qu'en parallèle il inhibe des enzymes de dégradation de la matrice extracellulaire (MMP3).

Et cet effet de l'extrait aqueux de fruit de rose (0.15% en poids de matière première équivalent à 25.5μg/mL(/MS) est beaucoup plus important que celui obtenu dans la même expérience pour un cryoextrait de fleurs à une dose plus de 2 fois supérieure (1% en poids de matière première équivalent à 65μg/mL(MS)) comme le montrent les résultats suivants :

Tableau 8

| Gène | Extrait* 0.15% | Cryoextrait** 1% | Vit C 50μg/mL (contrôle positif) |
|---|---|---|---|
| Collagène 1 | 16,015 | 7,998 | 3,471 |

(suite)

| Gène | Extrait* 0.15% | Cryoextrait** 1% | Vit C 50μg/mL (contrôle positif) |
|---|---|---|---|
| Collagène 3 | 2,818 | 1,928 | 2,599 |
| Collagène 5 | 12,987 | 2,960 | 1,911 |
| Elastine | 15,762 | 7,353 | 1,016 |
| Emilin 1 | 5,330 | 1,198 | 2,509 |
| Fibrillin 1 | 1,947 | 0,960 | 0,989 |
| Lysyl oxidase like 1 LOWL1 | 2,995 | 1,177 | 1,202 |
| Procollagen C endopeptidase enhancer | 1,450 | 0,888 | 1,156 |
| Matrix Metalloproeinase 3 | 0,679 | 0,471 | 1,205 |
| *extrait aqueux de fruits de rose décrit dans le paragraphe matériels et méthodes **extrait de fleurs de rose décrit dans le paragraphe matériels et méthodes | | | |

## Exemple 4: Effet de l'extrait aqueux de fruits de rose sur la perméabilité vasculaire (anti-cernes/ microcirculation)

Dans le cadre de l'étude TLDA décrite à l'exemple 1, il a également été montré que l'extrait de fruits de rose à la dose 0.15% inhibait de -74% l'expression du VEGF, facteur de croissance de l'endothélium vasculaire, impliqué dans la perméabilité vasculaire. Cet effet est particulièrement avantageux pour une utilisation de l'extrait selon l'invention dans la zone du contour des yeux.

L'ensemble de ces résultats montrent que l'extrait aqueux de fruits de rose selon l'invention, en particulier l'extrait aqueux de fruits de rose comprenant 1 % en poids de matière sèche (matière active), 97.5% en poids d'eau, 0.5% d'acide citrique et des conservateurs qsp100%, dès la dose de 0.15% :

- augmente la synthèse d'ATP favorisant l'apport d'énergie aux cellules de la peau ;
- stimule l'expression génique de marqueurs de la cohésion et de la différenciation épidermique participant au renforcement de la fonction barrière ;
- stimule l'expression géniques de protéines de la matrice extracellulaire et inhibe des enzymes de dégradation, participant à la densification de la matrice extracellulaire, et
- inhibe l'expression génique du facteur de croissance de l'endothélium vasculaire VEGF, participant à une diminution de la perméabilité vasculaire.

Et que ces effets stimulants sur la fonction barrière et la matrice extracellulaire sont plus importants que ceux obtenus avec un cryoextrait de fleurs.

On comprend donc l'intérêt d'utiliser un extrait de fruits de rose selon l'invention pour favoriser et/ou améliorer la cohésion et de la différenciation épidermique, le renforcement de la fonction barrière, la densification de la matrice extracellulaire, la diminution de la perméabilité cellulaire, au niveau de la peau du visage et en particulier dans la zone du contour des yeux, particulièrement fine et fragile et sujette à la formation de cernes et/ou poches.

L'extrait de fruits de rose selon l'invention a été mis en œuvre, à différentes concentrations, seul ou en combinaison avec d'autres extraits de rose aux propriétés complémentaires, dans différentes formulations cosmétiques Les pourcentages % sont exprimés en poids de matière première sauf indication contraire.

## Exemple 5 : Formulations cosmétiques

### 5.1 Composition sous la forme d'une émulsion

Phase aqueuse :

| | |
|---|---|
| Eau déminéralisée | qsp 100,0% |
| Glycols | 20,0% |
| Conservateurs | 0,6% |

(suite)

| | |
|---|---|
| Chélatant | 0,04% |
| Carbomer (Carbopol® 981) | 0,3% |
| Sodium polyacrylate (Covacryl® MV60) | 0,2% |
| Sodium Hydroxide | 0,15% |
| **Extrait aqueux de fruits de rose selon l'invention*** | **3,0%** |

Phase grasse :

| | |
|---|---|
| Huile végétale, esters, silicones | 16% |
| Anti-oxydant | 0,2% |
| Concentré de parfum | 0,4% |
| Steareth-2 | 0,8% |
| Steareth-21 | 1,5% |
| * tel que décrit dans le paragraphe matériel et méthodes | |

La composition est préparée selon le mode opératoire suivant :

- les gélifiants sont dispersés dans la phase aqueuse (hors extrait aqueux de fruits de rose et hydroxyde de sodium) qui est portée à 70°C ;
- la phase grasse (hors concentré de parfum, antioxydant) est chauffée à 70°C ;
- l'émulsion est réalisée par introduction de la phase grasse dans la phase aqueuse sous forte agitation ;
- les gélifiants sont neutralisés par ajout d'hydroxyde de sodium et l'émulsion est refroidie sous agitation modérée avec introduction du concentré de parfum, de l'antioxydant et de l'extrait aqueux de fruits de rose à basse température.

Appliquée sur la peau, en particulier du visage, cette composition apporte nutrition, fermeté et hydratation à la peau.

**5.2 Composition sous la forme d'une dispersion solide de corps gras, de forme sphérique ou sphéroïde**

Phase aqueuse

| | |
|---|---|
| Eau déminéralisée | qsp100% |
| Cryoextrait de rose* | 3.00% |
| **Extrait aqueux de fruits de rose selon l'invention*** | **3.00%** |
| Phenoxyethanol | 0.42% |
| Gomme de xanthane | 0.36% |

Phase grasse

| | |
|---|---|
| $C_{10-18}$ triglycerides | 38.84% |
| Satin Oil* | 1% |
| Anti Oxydant | 0.1% |
| *tels que décrits dans le paragraphe Matériel et méthodes ci-dessus. | |

La composition est préparée selon le mode opératoire suivant :

- on chauffe la cire (C10-18 triglycérides = Lipocire de Gatefossé) au-dessus de son point de fusion (quelques degrés) avec l'antioxydant et l'extrait huileux de rose Satin oil ;
- la phase grasse fondue est versée sous agitation dans de l'eau préalablement chauffée à la même température que la phase grasse ;
- .l'ensemble est maintenu sous agitation par un système rotatif pendant quelques minutes jusqu'à obtenir la taille de goutelettes souhaitée ;
- la dispersion obtenue est rapidement refroidie par addition d'eau glycolée préalablement refroidie (environ -4°C) pour solidifier les sphéroides lipidiques ;
- .l'agitation est arrêtée lorsque les sphéroïdes sont solidifiés puis sont récupérés à la surface ou filtrés ;
- on prépare la phase aqueuse en mélangeant l'eau, la gomme de xanthane, le conservateur et le cryoextrait de rose, et l'extrait aqueux de fruits de rose selon l'invention ;
- on récupère les sphéroïdes à la surface et on les incorpore dans le gel de xanthane contenant le cryoextrait de rose et l'extrait aqueux de fruits de rose selon l'invention.

Appliquée sur la peau, cette composition confère un aspect nourri, plus lisse et plus ferme à la peau.

**5.3 : Composition sous la forme d'un gel aqueux pour le visage**

**Extrait aqueux de fruits de rose**

| | |
|---|---|
| **selon l'invention *** | **95.00%** |
| Conservateurs | 0,50% |
| Sucre | 0,20% |
| Carbomer | 0,70% |
| Eau purifiée | Qsp 100% |
| EDTA tetrasodique poudre | 0,20% |
| Sodium hydroxide | 0,17% |
| * tel que décrit dans le paragraphe Matériel et méthodes | |

L'extrait de fruits de rose selon l'invention et les conservateurs sont mélangés et homogénéisés à température ambiante sous agitation. Les sucres sont ajoutés sous agitation, puis le carbomer, puis l'eau et l'EDTA sous agitation. Le gel aqueux est ensuite neutralisé avec l'ajout d'hydroxyde de sodium sous agitation jusqu'à l'obtention d'un gel homogène.
Appliqué sur la peau du visage, ce gel aqueux confère un effet frais, et apporte souplesse et fermeté à la peau.

**5.4: Composition sous la forme d'un gel aqueux**

| | |
|---|---|
| **Extrait aqueux de fruits de rose selon l'invention*** | **80.00%** |
| Phenoxyéthanol | 0,50% |
| Glycols | 12.0% |
| Polyol | 2.0% |
| Polymère épaississant (Nom INCI Hydroxyethyl Acrylate/Sodium Acryloyldimethyl Taurate Copolymer, Sepinov™ EMT 10 | 1,60% |
| BHT | 0,20% |
| Alcool à 96,2% vol kg | Qsp100% |
| Parfum de rose | 0.30% |
| * tel que décrit dans le paragraphe Matériel et méthodes | |

L'extrait aqueux de fruits de rose selon l'invention, le phenoxyéthanol, et les glycols sont mélangés sous agitation à température ambiante ; le polyol est ensuite ajouté sous agitation. Le tensioactif Sepinov™ EMT 10 est ensuite ajouté et émulsionné dans la phase aqueuse sous agitation et l'on ajoute ensuite et l'alcool, le BHT et le parfum de rose.

Appliqué sur la peau du visage, ce gel aqueux confère un effet frais, et apporte souplesse et fermeté à la peau.

### 5.5 : Composition sous la forme d'un gel-sérum micronutritif pour le contour des yeux

| | |
|---|---|
| Eau purifiée | Qsp 100.00% |
| Glycols | 13,0% |
| Conservateurs | 0,60% |
| Carbomer | 0,80% |
| Glyceryl stearate citrate | 0,70% |
| Lécithine et sodium acrylates copolymer (Lecigel PCR négatif) | 1,20% |
| Isostearate isostearyle | 9,0% |
| Bis-diglyceryl polyacyladipate-2 (Softisan 649 MB) | 1,0% |
| Silice | 2,0% |
| Nacres | 1,0% |
| Cryoextrait de rose* | 3.0% |
| Extrait de *Centella asiatica* | 0,5% |
| Extrait de marron d'inde | 0,1% |
| **Extrait aqueux de fruit de rose selon l'invention** * | 3,0% |
| Tocopheryl acetate | 0,10% |
| Parfum de rose | 0,20% |
| Satin oil* | 1.0% |
| * tels que décrits dans le paragraphe Matériels et Méthodes | |

Les ingrédients de la phase aqueuse (l'eau, les glycols et le carbomer) sont mélangés à 80°C sous agitation. On ajoute ensuite les conservateurs, puis les gélifiants à 80°C sous agitation puis la température est abaissée à 75°C. Puis les tensioactifs sont émulsionnés dans la phase aqueuse sous agitation. Les charges et nacres, préalablement empâtées et homogénéisées, sont ajoutées à 40°C. Sont ensuite ajoutés à 40°C le cryoextrait, l'extrait aqueux de rose selon l'invention et les autres extraits, sous agitation jusqu'à 30°C.

Après application sur le contour des yeux de ce gel-sérum, les poches et les cernes semblent s'estomper pour un regard visiblement défatigué, illuminé, éclatant de fraîcheur. La peau apparaît nourrie, plus ferme, plus lisse (diminution de l'apparence des rides et ridules), hydratée et de couleur plus homogène.

## Revendications

1. Extrait aqueux de fruits de rose de la variété Evanrat ou rose Jardin de Granville® obtenu au moyen d'un solvant polaire cosmétiquement acceptable.

2. Extrait aqueux de fruits de rose de la variété Evanrat ou rose Jardin de Granville® selon la revendication 1, **caractérisé en ce qu'**il comprend de 1 à 2 % en poids de fruits de rose (matière active), 97.5% à 98.5% d'eau, en particulier 1% de fruits de rose (matière active), 97.5% d'eau, 0.5% d'acide citrique et des conservateurs qs 100%.

3. Extrait aqueux de fruits de rose de la variété Evanrat ou rose Jardin de Granville® selon la revendication 1 comprenant une teneur en sucres totaux allant de 1 à 3%, et comprenant notamment du fructose, du glucose et éventuellement du saccharose.

4. Extrait aqueux de fruits de rose de la variété Evanrat ou rose Jardin de Granville® selon la revendication 1, obtenu selon le procédé d'extraction comprenant les étapes suivantes :

1. récolte des fruits de rose frais, éventuellement stockés à -20°C,
2. broyage des fruits
3. mélange de 5 à 30%, notamment 10% de matériel végétal dans de l'eau de préférence acidifiée à une température allant notamment de 10 à 60°C, en particulier 30°C,
4. montée progressive en température du mélange, en particulier de 30 à 100°C, notamment jusqu'à 50 à 70°C, voire jusqu'à 60°C,
5. extraction éventuellement sous agitation,
6. séparation liquide/solide,
7. optionnellement ajout de conservateurs,
8. filtrations, en particulier jusqu'à 0,22$\mu$m,
9. optionnellement conditionnement, le cas échéant sous azote,
10. optionnellement stockage à +4°C.

**5.** Composition cosmétique pour application topique sur la peau comprenant, dans un milieu physiologiquement acceptable, au moins une quantité efficace d'au moins un extrait aqueux de fruits de rose de la variété Evanrat ou rose Jardin de Granville® tel que défini dans l'une quelconque des revendications 1 à 4.

**6.** Composition cosmétique selon la revendication 5, **caractérisée en ce que** l'extrait aqueux de fruits de rose est présent dans la composition en une teneur allant de 0,1% à 95%, en particulier de 0,1% à 20%, de préférence de 0,5% à 10% et de préférence encore de 1% à 5% en poids de matière première par rapport au poids total de ladite composition.

**7.** Composition cosmétique selon l'une des revendications 5 ou 6, **caractérisée en ce que** la composition cosmétique est sous la forme d'une solution, d'une émulsion, d'un sérum ou d'un gel, de préférence un gel aqueux pour le contour des yeux.

**8.** Procédé cosmétique non thérapeutique destiné à favoriser et/ou améliorer l'apport en nutriments à la peau, favoriser et/ou améliorer la cohésion et/ou la différenciation épidermique, favoriser et/ou améliorer la fonction barrière, densifier la matrice extracellulaire et/ou diminuer sa dégradation, améliorer l'élasticité et/ou la fermeté de la peau en particulier du contour des yeux, prévenir et/ou diminuer les cernes et/ou les poches du contour des yeux, améliorer l'éclat et/ou l'homogénéité de la peau en particulier du contour des yeux, favoriser et/ou améliorer l'hydratation, et/ou prévenir et/ou diminuer la formation des rides et/ou ridules, comprenant l'application sur la peau, en particulier du visage et/ou du cou et de préférence du contour des yeux, d'une composition cosmétique telle que définie dans l'une quelconque des revendications 5 à 7.

**9.** Procédé cosmétique selon la revendication 8, destiné à prévenir et/ou diminuer les cernes et/ou les poches du contour des yeux, et/ou améliorer l'éclat et/ou l'homogénéité de la peau du contour des yeux, comprenant l'application sur le contour des yeux d'une composition cosmétique telle que définie dans l'une quelconque des revendications 5 à 7.

**10.** Utilisation cosmétique non thérapeutique d'au moins une quantité efficace d'au moins un extrait aqueux de fruits de rose de la variété Evanrat ou rose Jardin de Granville® tel que défini dans l'une quelconque des revendications 1 à 4, comme agent destiné à favoriser et/ou améliorer l'apport en nutriments à la peau, favoriser et/ou améliorer la cohésion et/ou la différenciation épidermique, favoriser et/ou améliorer la fonction barrière, densifier la matrice extracellulaire et/ou diminuer sa dégradation, améliorer l'élasticité et/ou la fermeté de la peau en particulier du contour des yeux, prévenir et/ou diminuer les cernes et/ou les poches du contour des yeux, améliorer l'éclat et/ou l'homogénéité de la peau en particulier du contour des yeux, favoriser et/ou améliorer l'hydratation, et/ou prévenir et/ou diminuer la formation des rides et/ou ridules.

**11.** Ensemble cosmétique comprenant :

- Une composition cosmétique telle que définie dans l'une quelconque des revendications 5 à 7, et
- Un applicateur adapté à une application topique sur la peau du visage, en particulier sur le contour des yeux.

**Patentansprüche**

1. Wässriger Extrakt aus Rosenfrüchten der Sorte Evanrat oder Rose Jardin de Granville®, der mit Hilfe eines polaren kosmetisch akzeptablen Lösungsmittels gewonnen wurde.

2. Wässriger Extrakt aus Rosenfrüchten der Sorte Evanrat oder Rose Jardin de Granville® nach Anspruch 1, **dadurch gekennzeichnet, dass** er 1 bis 2 Gew.-% Rosenfrüchte (Wirkstoff), 97,5% bis 98,5% Wasser, vor allem 1% Rosenfrüchte (Wirkstoff), 97,5% Wasser, 0,5% Zitronensäure und Konservierungsstoffe qs 100% umfasst.

3. Wässriger Extrakt aus Rosenfrüchten der Sorte Evanrat oder Rose Jardin de Granville® nach Anspruch 1, umfassend einen Zuckergehalt insgesamt von 1 bis 3% und umfassend insbesondere Fruktose, Glukose und eventuell Saccharose.

4. Wässriger Extrakt aus Rosenfrüchten der Sorte Evanrat oder Rose Jardin de Granville® nach Anspruch 1, erhalten gemäß dem Extraktionsverfahren, das die folgenden Schritte umfasst:

   1. Ernten frischer Rosenfrüchte, eventuell gelagert bei -20°C,
   2. Zerkleinern der Früchte,
   3. Mischen von 5 bis 30%, insbesondere 10% pflanzlichen Materials in vorzugsweise angesäuertem Wasser bei einer Temperatur von insbesondere 10 bis 60°C, vor allem 30°C,
   4. schrittweises Anheben der Temperatur des Gemischs, vor allem von 30 auf 100°C, insbesondere bis 50 bis 70°C, ja sogar 60°C,
   5. Extrahieren, eventuell unter Rühren,
   6. Trennen Flüssigkeit/Feststoff,
   7. optional Hinzufügen von Konservierungsstoffen,
   8. Filtrieren, vor allem bis 0,22 $\mu$m,
   9. optional Verpacken, gegebenenfalls unter Stickstoff,
   10. optional Lagern bei +4°C.

5. Kosmetische Zusammensetzung für eine topische Anwendung auf der Haut, umfassend, in einem physiologisch akzeptablen Medium, mindestens eine wirksame Menge mindestens eines wässrigen Extrakts aus Rosenfrüchten der Sorte Evanrat oder Rose Jardin de Granville® nach einem der Ansprüche 1 bis 4.

6. Kosmetische Zusammensetzung nach Anspruch 5, **dadurch gekennzeichnet, dass** der wässrige Extrakt aus Rosenfrüchten in der Zusammensetzung in einem Gehalt vorhanden ist, der von 0,1% bis 95%, vor allem von 0,1% bis 20%, vorzugsweise von 0,5% bis 10% und noch vorzugsweiser von 1% bis 5 Gew.-% des Rohstoffs im Verhältnis zum Gesamtgewicht der Zusammensetzung reicht.

7. Kosmetische Zusammensetzung nach einem der Ansprüche 5 oder 6, **dadurch gekennzeichnet, dass** die kosmetische Zusammensetzung in Form einer Lösung, einer Emulsion, eines Serums oder eines Gels, vorzugsweise eines wässrigen Gels für die Augenpartie, vorliegt.

8. Nicht-therapeutisches kosmetisches Verfahren, das bestimmt ist, die Nährstoffzufuhr in die Haut zu fördern und/oder zu verbessern, die Kohäsion und/oder die Differenzierung der Epidermis zu fördern und/oder zu verbessern, die Barrierefunktion zu fördern und/oder zu verbessern, die extrazelluläre Matrix zu verdichten und/oder deren Zerfall zu verringern, die Elastizität und/oder die Straffheit der Haut, vor allem der Augenpartie, zu verbessern, den Augenringen und/oder den Tränensäcken in der Augenpartie vorzubeugen und/oder diese zu verringern, das Strahlen und/oder die Ebenmäßigkeit der Haut, vor allem der Augenpartie, zu verbessern, die Feuchtigkeitsversorgung zu fördern und/oder zu verbessern, und/oder der Bildung von Falten und/oder Fältchen vorzubeugen und/oder diese zu verringern, umfassend das Auftragen auf die Haut, vor allem des Gesichts und/oder des Halses und vorzugsweise der Augenpartie, einer kosmetischen Zusammensetzung nach einem der Ansprüche 5 bis 7.

9. Kosmetisches Verfahren nach Anspruch 8, das bestimmt ist, den Augenringen und/oder den Tränensäcken in der Augenpartie vorzubeugen und/oder diese zu verringern, und/oder das Strahlen und/oder die Ebenmäßigkeit der Haut der Augenpartie zu verbessern, umfassend das Auftragen auf die Augenpartie einer kosmetischen Zusammensetzung nach einem der Ansprüche 5 bis 7.

10. Nicht-therapeutische kosmetische Verwendung mindestens einer wirksamen Menge mindestens eines wässrigen Extrakts aus Rosenfrüchten der Sorte Evanrat oder Rose Jardin de Granville® nach einem der Ansprüche 1 bis 4 als Mittel, das bestimmt ist, die Nährstoffzufuhr in die Haut zu fördern und/oder zu verbessern, die Kohäsion und/oder die Differenzierung der Epidermis zu fördern und/oder zu verbessern, die Barrierefunktion zu fördern und/oder zu verbessern, die extrazelluläre Matrix zu verdichten und/oder deren Zerfall zu verringern, die Elastizität und/oder die Straffheit der Haut, vor allem der Augenpartie, zu verbessern, den Augenringen und/oder den Tränensäcken in der Augenpartie vorzubeugen und/oder diese zu verringern, das Strahlen und/oder die Ebenmäßigkeit der Haut, vor allem der Augenpartie, zu verbessern, die Feuchtigkeitsversorgung zu fördern und/oder zu verbessern, und/oder der Bildung von Falten und/oder Fältchen vorzubeugen und/oder diese zu verringern.

**11.** Kosmetische Gesamtheit, umfassend:

- eine kosmetische Zusammensetzung nach einem der Ansprüche 5 bis 7, und
- einen Applikator, der für eine topische Anwendung auf der Haut des Gesichts, vor allem auf der Augenpartie, geeignet ist.

**Claims**

1. An aqueous extract of rose fruit of the Evanrat or Jardin de Granville® rose variety obtained using a cosmetically acceptable polar solvent.

2. The aqueous extract of rose fruit of the Evanrat or Jardin de Granville® rose variety according to claim 1, **characterised in that** it comprises 1 to 2% by weight rose fruit (active ingredient), 97.5% to 98.5% water, in particular 1% rose fruit (active ingredient), 97.5% water, 0.5% citric acid and preservatives qs 100%.

3. The aqueous extract of rose fruit of the Evanrat or Jardin de Granville® rose variety according to claim 1, comprising a total sugar content ranging from 1 to 3%, and notably comprising fructose, glucose and possibly sucrose.

4. The aqueous extract of rose fruit of the Evanrat or Jardin de Granville® rose variety according to claim 1, obtained according to the extraction process comprising the following steps:

   1. harvesting fresh rose fruit, optionally stored at -20°C,
   2. grinding the fruit
   3. mixing 5 to 30%, in particular 10% of plant material in preferably acidified water at a temperature ranging notably from 10 to 60°C, in particular 30°C,
   4. gradual increasing of the temperature of the mixture, in particular from 30 to 100°C, notably up to 50 to 70°C, or even up to 60°C,
   5. extraction optionally under stirring,
   6. liquid/solid separation,
   7. optional addition of preservatives,
   8. filtration, especially to 0.22 $\mu$m,
   9. optionally packaging, under nitrogen if necessary,
   10. optionally storage at +4°C.

5. A cosmetic composition for topical application on the skin, comprising, in a physiologically acceptable medium, at least an effective quantity of at least one aqueous extract of rose fruit of the Evanrat or Jardin de Granville® varieties such as defined in any one of claims 1 to 4.

6. The cosmetic composition according to claim 5, **characterised in that** the aqueous extract of rose fruit is present in the composition in a content ranging from 0.1% to 95%, in particular from 0.1% to 20%, preferably from 0.5% to 10% and more preferably from 1% to 5% by weight of raw material based on the total weight of said composition.

7. The cosmetic composition according to claims 5 or claim 6, **characterised in that** the cosmetic composition is in the form of a solution, an emulsion, a serum or a gel, preferably an aqueous gel for the eye contour area.

8. A non-therapeutic cosmetic process intended to promote and/or improve the supply of nutrients to the skin, promote and/or improve epidermal cohesion and/or differentiation, promote and/or improve the barrier function, densify the extracellular matrix and/or reduce its degradation, improve the elasticity and/or firmness of the skin, in particular of the eye contour area, prevent and/or reduce dark circles and/or bags around the eyes, improve the radiance and/or homogeneity of the skin, in particular the eye contour area, promote and/or improve hydration, and/or prevent and/or reduce the formation of wrinkles and/or fine lines, comprising the application to the skin, in particular the face and/or neck and preferably the eye contour area, of a cosmetic composition as defined in any one of claims 5 to 7.

9. The cosmetic process according to claim 8, for preventing and/or reducing dark circles and/or bags around the eyes and/or improving the radiance and/or homogeneity of the skin around the eyes, comprising the application to the eye contour area of a cosmetic composition as defined in any one of claims 5 to 7.

10. A non-therapeutic cosmetic use of at least one effective amount of at least one aqueous extract of rose fruit of the Evanrat or Jardin de Granville® rose variety, as defined in any one of claims 1 to 4, as an agent intended to promote and/or improve the supply of nutrients to the skin, promote and/or improve epidermal cohesion and/or differentiation, promote and/or improve the barrier function, densify the extracellular matrix and/or reduce its degradation, improve the elasticity and/or firmness of the skin, particularly around the eyes, prevent and/or reduce dark circles and/or bags around the eyes, improve the radiance and/or homogeneity of the skin, particularly around the eyes, promote and/or improve hydration, and/or prevent and/or reduce the formation of wrinkles and/or fine lines.

11. A cosmetic set comprising:

- A cosmetic composition as defined in any one of claims 5 to 7, and
- An applicator suitable for topical application to the skin of the face, especially around the eyes.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 0425391 A

- WO 2010112760 A

**Littérature non-brevet citée dans la description**

- **PARK K.** Role of micronutrients in skin health and function. *Biomol.Ther.,* 2015, vol. 23, 207-217
- **PARK K.** Role of micronutrients in skin health and function. *Biomol.Ther.,* 2015, vol. 23, 207-217
- **POLEFKA T.** Interaction of minerai salts with the skin : a literature survey. *Int J. Cosmetic. Sci.,* 2012, vol. 34, 416-423

- **BOELSMA E.** Nutritiaonal skin care : health effects of micronutrients and fatty acids. *Am. J. Clin. Nutr.,* 2001, vol. 73, 853-864
- **WINKLER P.** Minerais and the skin in Nutrition and the skin-Lessons for anti-aging, beauty and healthy skin. 2011, 91-109